# EUROPÄISCHE PATENTSCHRIFT

(11) **EP 2 060 299 B1**
(45) Veröffentlichungstag und Bekanntmachung des Hinweises auf die Patenterteilung: **07.01.2015**
(21) Anmeldenummer: 08166101.9
(22) Anmeldetag: 08.10.2008
(51) Int. Cl.: A61N 1/362, A61N 1/368

(54) **Biventrikulärer Herzstimulator**
Biventricular heart stimulator
Stimulateur cardiaque bi-ventriculaire

(30) Priorität: 14.11.2007 DE 102007054178
(43) Veröffentlichungstag der Anmeldung: 20.05.2009
(73) Patentinhaber: Biotronik CRM Patent AG, 6341 Baar (CH)
(72) Erfinder: Dörr, Thomas, 12437, Berlin (DE); Müssig, Dirk, West Linn, 97068 (US); Lang, Volker, 97068, West Linn (DE)
(74) Vertreter: Lindner-Vogt, Karin L.

(56) Entgegenhaltungen:
- US-A1- 2004 215 245
- US-A1- 2004 215 257
- US-A1- 2007 150 015
- US-B1- 7 043 301

## Beschreibung

Die Erfindung betrifft einen implantierbaren Herzstimulator für die kardiale Resynchronisationstherapie (CRT) eines Herzens. Der Herzstimulator kann ein Herzschrittmacher oder ein implantierbarer Kardioverter/Defibrillator (ICD) oder eine Kombination aus beidem sein, der in der Lage ist, beide Ventrikel eines Herzens zu stimulieren.

Typischerweise weist ein derartiger Herzstimulator wenigstens eine rechtsventrikuläre Sensingeinheit und eine rechtsventrikuläre Stimulationseinheit sowie eine linksventrikuläre Sensingeinheit und eine linksventrikuläre Stimulationseinheit auf. Diese Einheiten sind im Betrieb des Herzstimulators jeweils über Elektrodenleitungen mit an geeigneten Stellen im Herzen zu implantierenden Elektroden verbunden. Die Elektrodenleitung mit den Elektroden zum Erfassen elektrischer Potentiale im linken Ventrikel des Herzens sowie zur Abgabe linksventrikulärer Stimulationsimpulse sind typischerweise Bestandteil einer linksventrikulären Elektrodenleitung, die durch den Koronarsinus eines Herzens verlegt wird und daher auch als Koronarsinuselektrodenleitung bezeichnet wird. Die Elektroden zum Erfassen elektrischer Potentiale im rechten Ventrikel sowie zur Abgabe rechtsventrikulärer Stimulationsimpulse sind typischerweise an einer rechtsventrikulären Elektrodenleitung befestigt, deren distales Ende bis in den Apex des rechten Ventrikels ragt. Die Elektrodenleitungen sind typischerweise an ihrem proximalen Ende über normierte Steckverbindungen mit einem entsprechenden Herzstimulator verbunden.

Die typischen Stimulationsmodi eines rechtsventrikulären Herzstimulators wie beispielsweise VVI, VVD oder DDD können als bekannt vorausgesetzt werden. Gleiches gilt für die Abgabe von Stimulationsimpulsen nur im Bedarfsfall (Demand-Schrittmacher), bei der die Abgabe eines Stimulationsimpulses an eine jeweilige Kammer eines Herzens dann unterbunden wird, wenn zuvor in einem entsprechenden Escape-Intervall eine jeweilige Eigenaktion (intrinsische Kontraktion) der jeweiligen Herzkammer über eine dieser Herzkammer zugeordnete Sensingeinheit des Herzstimulators erfasst wurde. Diese an sich bekannten Konzepte können auch bei dem hier beschriebenen Herzstimulator verwirklicht sein.

Der hier betroffene Herzstimulator ist ein biventrikulärer Herzstimulator, der grundsätzlich in der Lage ist, beide Ventrikel des Herzens ständig oder bei Bedarf zu stimulieren.

So ist beispielsweise aus der US2004/0215257A1 ein System bekannt, das zwischen einer Stimulation des rechten Ventrikels oder einer biventrikulären Stimulation umschalten kann. Die US2004/0215245A1 beschreibt einen Defibrillator, der in der Lage ist biventrikuläre Stimulationsimpulse abzugeben.

Die Erfindung betriftt einen Herzstimulator, wie es durch Anspruch 1 definiert ist.

Ziel der Erfindung ist es, einen Herzstimulator zu schaffen, der selbstständig zwischen Ziel der Erfindung ist es, einen Herzstimulator zu schaffen, der selbstständig zwischen einem biventrikulären Stimulationsmodus für die kardiale Resynchronisationstherapie und einem rechtsventrikulären Stimulationsmodus - im Folgenden auch Normalmodus genannt - wechselseitig umschalten kann. Je nach Ausführungsform des Herzstimulators ist dieser dann so ausgebildet, in dem Normalmodus, in dem nur der rechte Ventrikel und dieser gegebenenfalls nur im Bedarfsfall stimuliert wird, in den an sich bekannten Modi zu operieren, d. h. beispielsweise auch in einem Hysteresemodus wie beispielsweise der Frequenzhysterese oder der AV-Zeit-Hysterese oder in einem VP-Supressionsmodus, in dem die Abgabe ventrikulärer Stimulationsimpulse grundsätzlich inhibiert wird.

Erfindungsgemäß wird die Aufgabe, einen Herzstimulator zu schaffen, der selbstständig zwischen einem biventrikulären Stimulationsmodus und einem rechtsventrikulären Stimulationsmodus hin- und herschaltet, dadurch gelöst, dass der Herzschrittmacher neben bekannten rechts- und linksventrikulären Sensingeinheiten und Stimulationseinheiten eine Stimulationssteuereinheit aufweist, die ausgebildet ist, den Herzstimulator immer dann in den rechtsventrikulären Stimulationsmodus zu schalten, in dem keine linksventrikulären Stimulationsimpulse abgegeben werden, wenn die Dauer eines jeweils aktuellen QRS-Signalabschnitts kürzer ist, als ein erster Referenzwert, und in einen biventrikulären Stimulationsmodus zu schalten, in dem sowohl der rechte als auch der linke Ventrikel stimuliert werden können, wenn die Dauer eines jeweils aktuellen QRS-Signalabschnittes länger ist als ein vorgegebener zweiter Vergleichswert. Der erste und der zweite Referenzwert können dabei identisch sein. Die Dauer des QRS-Signalabschnittes bezeichnet die Dauer des an sich bekannten QRS-Komplexes in einem Elektrokardiogramm.

Vorzugsweise ist der Herzstimulator dazu ausgebildet, ein die Dauer des jeweiligen QRS-Signalabschnittes repräsentierendes Signal aus einem jeweiligen intrinsischen VV-Intervall abzuleiten, welches die Zeitdauer zwischen einer von der rechtsventrikulären Sensingeinheit erfassten rechtsventrikulären Kontraktion und der zugehörigen, von einer linksventrikulären Sensingeinheit erfassten linksventrikulären Kontraktion des Herzens repräsentiert.

Alternativ dazu kann der Herzstimulator auch ausgebildet sein, das die Dauer des QRS-Signalabschnittes repräsentierende Signal auch in Abhängigkeit einer jeweils erfassten atrioventrikulären Überleitungszeit (AV-Intervall) zu bestimmen. Dies kann entweder die Dauer zwischen einer erfassten rechtsatrialen Kontraktion und der zugehörigen rechtsventrikulären Kontraktion oder die Dauer zwischen einer erfassten rechtsatrialen Kontraktion und der zugehörigen linksventrikulären Kontraktion sein. Hierzu weist der Herzstimulator zusätzlich wenigstens eine atriale Sensingeinheit auf. Ist der Herzstimulator außerdem zur Stimulation des rechten Atriums ausgebildet, weist er außerdem noch eine rechtsatriale Stimulationseinheit auf.

Ein Vorteil eines derartigen biventrikulären Herzstimulators besteht darin, dass er den Einsatzbereich von Herzstimulatoren auch auf solche Patienten erweitert, die bisher nur ungenügend behandelt werden können. Immer dann, wenn Patienten nur gelegentlich die Symptome zeigen, die den Einsatz eines biventrikulären Herzstimulators im biventrikulären Stimulationsmodus indizieren, ist es wünschenswert, diesen Stimulationsmodus anbieten zu können und gleichzeitig aber auch den reinen rechtsventrikulären Stimulationsmodus zur Verfügung zu stellen. Es hat sich nämlich gezeigt, dass dem reinen rechtsventrikulären Stimulationsmodus grundsätzlich der Vorzug zu geben ist. Die biventrikuläre Stimulation kann nämlich zu einer Normalisierung des Herzrhythmus führen, in dem dann eine biventrikuläre Stimulation nicht mehr vorteilhaft ist. Auch profitieren Patienten mit einem intermittierenden Links-Schenkelblock von einem biventrikulären Herzstimulator, der automatisch zwischen einem biventrikulären Stimulationsmodus und einem rechtsventrikulären Stimulationsmodus hin- und herschalten kann. Als weiterer Vorteil kommt hinzu, dass die dauerhafte Stimulation des rechten und des linken Ventrikels wesentlich mehr Energie erfordert, als die Stimulation allein des rechten Ventrikels und dann auch nur im Bedarfsfall. Das automatische Umschalten in den rechtsventrikulären Stimulationsmodus trägt somit zu einem energieeffizienteren Betrieb des Herzstimulators bei und verlängert damit dessen Lebensdauer.

Bisherige Herzstimulatoren erlauben nur ein manuelles Umschalten zwischen einem biventrikulären Stimulationsmodus und einem rechtsventrikulären Stimulationsmodus durch einen Arzt.

Vorteilhafte Ausgestaltungen des erfindungsgemäßen Herzstimulators betreffen Details der automatischen Umschaltung. Beispielsweise können für die automatische Umschaltung wie bereits angedeutet zwei unterschiedliche Referenzwerte vorgesehen sein, so dass sich eine Hysterese ergibt oder es kann jeweils für das Umschalten von dem rechtsventrikulären in den biventrikulären Stimulationsmodus und umgekehrt der gleiche Referenzwert vorgesehen sein. Außerdem kann der Herzstimulator entweder ausgebildet sein, ein Umschalten in den jeweils anderen Stimulationsmodus jeweils bei einmaligen Über- oder Unterschreiten des jeweiligen Referenzwertes vorzunehmen. Alternativ und bevorzugt ist der Herzstimulator jedoch dazu ausgebildet, ein Umschalten erst dann vorzunehmen, wenn der jeweilige Referenzwert für eine vorgegebene Anzahl von Herzzyklen hintereinander über- bzw. unterschritten wird.

Weitere vorteilhafte Ausgestaltungen betreffen das Vorsehen eines VP-Supressionsmodus sowie die Speicherung der Zeitpunkte eines jeweiligen Umschaltens zwischen einem der Stimulationsmodi in einer entsprechenden Speichereinheit. Vorzugsweise ist diese Speichereinheit mit einer Telemetrieeinheit verbunden, die es erlaubt, die in der Speichereinheit gespeicherten Daten drahtlos an ein externes Gerät und von dort aus gegebenenfalls weiter bis zu einem zentralen Servicecenter zu übertragen. Auf diese Weise könnten die Umschaltzeitpunkte zwischen den Stimulationsmodi beispielsweise fernabgefragt werden.

Weiterhin kann die automatische Umschaltung zwischen den Stimulationsmodi (rechtsventrikulär und biventrikulär) auch in Abhängigkeit weiterer, vom Herzstimulator zu erfassenden Größen, wie beispielsweise der an sich bekannten Ejektionsfraktion oder des mitralen Rückflusses erfolgen. Vorzugsweise ist die Stimulationssteuereinheit ausgebildet, von einer rechtsventrikulären Stimulation zu einer biventrikulären Stimulation umzuschalten, wenn entweder die gemessene Ejektionsfraktion einen programmierbaren Schwellwert unterschreitet oder der mitrale Rückfluss einen programmierbaren Schwellwert überschreitet. Werden beide Parameter vom Herzstimulator gleichzeitig ausgewertet, erfolgt die Umschaltung von einem rechtsventrikulären Modus in einen biventrikulären Modus, wenn entweder die Ejektionsfraktion den programmierten Schwellwert unterschreitet oder der mitrale Rückfluss den programmierten Schwellwert überschreitet. Die Umschaltung von einem biventrikulären in einen rechtsventrikulären Stimulationsmodus erfolgt dann, wenn die gemessenen Ejektionsfraktion über einem programmierten zweiten (Hysterese) Schwellwert für die Ejektionsfraktion liegt und der mitrale Rückfluss gleichzeitig unter einem programmierten zweiten (Hysterese) Schwellwert für den mitralen Rückfluss liegt.

Weitere vorteilhafte Ausgestaltungen ergeben sich durch Kombination der hier beschriebenen Merkmale untereinander und mit solchen Merkmalen, die aus dem Stand der Technik bekannt sind.

Die Erfindung soll nun anhand eines Ausführungsbeispiels unter Bezug auf die Figuren näher erläutert werden. Diese zeigen in
- Figur 1:: eine schematische Darstellung eines Herztherapiesystems;
- Figur 2:: eine Darstellung eines Herzstimulators mit angeschlossenen, im Herzen angeordneten Elektroden;
- Figur 3:: ein schematisches Blockschaltbild eines Herzstimulators;
- Figur 4:: ein schematisches Blockschaltbild zur näheren Erläuterung des Herzstimulators vor dem Hintergrund des Blockschaltbildes aus Figur 3;
- Figur 5:: ein Blockschaltbild einer alternativen Ausführungsvariante;
- Figur 6:: ein Blockschaltbild einer weiteren alternativen Ausführungsvariante;
- Figur 7:: ein Flussdiagramm zur Erläuterung der Funktionsweise des Herzstimulators;
- Figur 8:: ein Flussdiagramm einer bevorzugten Betriebsweise des Herzstimulators und
- Figur 9:: ein Flussdiagramm zur Erläuterung einer weiteren alternativen Betriebsweise des Herzstimulators.

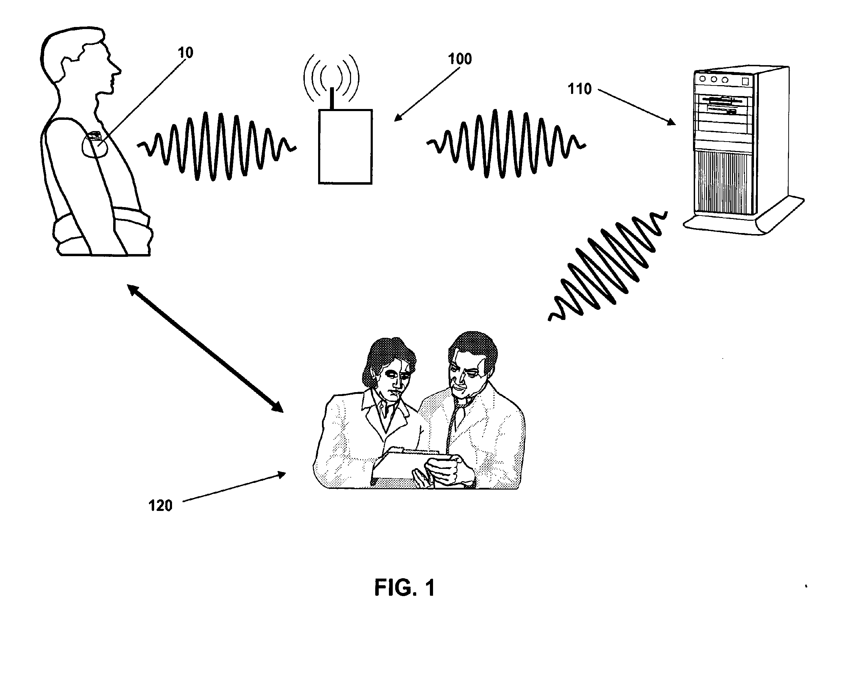

In Figur 1 ist zur Übersicht ein Herztherapiesystem dargestellt, dass neben einem implantierten Herzschrittmacher 10 ein externes Gerät (Patientengerät) 100 sowie ein durch einen Server symbolisch dargestelltes Servicecenter 110 umfasst. Der implantierbare Herzstimulator 10 besitzt eine Telemetrieeinheit, für die er drahtlos Daten mit dem externen Gerät 100 austauschen kann. Das externe Gerät 100 ist beispielsweise drahtgebunden mit dem Servicecenter 110 verbunden, so dass insgesamt Daten zwischen dem Servicecenter 110 und dem implantierbaren Herzstimulator 10 über das externe Gerät 100 als Relaisstation ausgetauscht werden können. Ein Ärzteteam 120 kann über einen datentechnischen Zugriff auf das Servicecenter 110 Einsicht in Daten nehmen, die das Servicecenter 110 von dem implantierbaren Herzstimulator 10 erhalten hat.

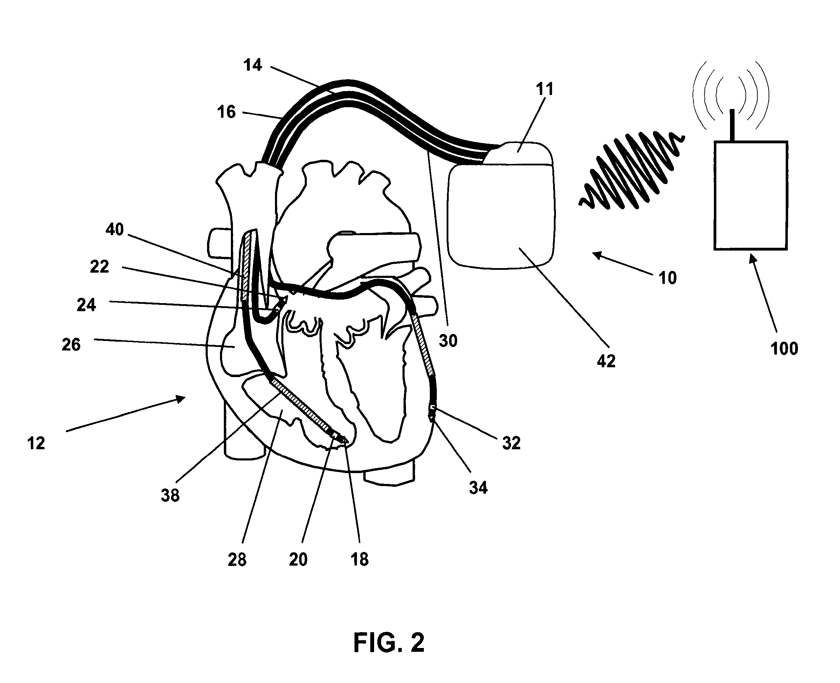

Figur 2 zeigt den implantierbaren Herzstimulator 10 in Form eines Dreikammerherzschrittmachers/Kardioverter/Defibrillators mit daran angeschlossenen Elektrodenleitungen 14, 16 und 30 in Verbindung mit einem Herz 12. Außerdem ist noch einmal das externe Gerät 100 in der Nähe des implantierbaren Herzstimulators 10 dargestellt. Die Elektrodenleitungen 14, 16 und 30 sind über bekannte, standardisierte Steckverbindungen mit Kontaktbuchsen in einem Header (Anschlussgehäuse) 11 des Herzstimulators 10 elektrisch verbunden. Auf diese Weise sind die Elektrodenleitungen 14, 16 und 30 auch mit elektronischen Komponenten im inneren eines hermetisch dichten Metallgehäuses 42 des Herzstimulators 10 verbunden. Diese Komponenten sind nachfolgend detaillierter schematisch dargestellt und bestimmen die Funktionsweise des Herzstimulators 10.

Die Elektrodenleitung 14 ist eine rechtsatriale Elektrodenleitung und besitzt an ihrem distalen Ende eine atriale Spitzenelektrode RA Tip 22 sowie in kurzer Entfernung davon eine atriale Ringelektrode RA Ring 24, die beide im rechten Atrium 26 des Herzens 12 platziert sind.

Die Elektrodenleitung 16 ist eine rechtsventrikuläre Elektrodenleitung und besitzt an ihrem distalen Ende eine rechtsventrikuläre Spitzenelektrode RV Tip 18 und in unmittelbarer Nähe eine rechtsventrikuläre Ringelektrode RV Ring 20. Beide Elektroden sind im Apex des rechten Ventrikels 28 des Herzens 12 angeordnet.

Außerdem ist die rechtsventrikuläre Elektrodenleitung 16 noch eine rechtsventrikuläre Schockwendel RV Schock 38 als großflächige Elektrode zur Abgabe von Relationsschocks. Eine weitere Schockwendel 40 ist in der Vena Cava Superior angeordnet und wird deshalb nachfolgend auch als SVC-Schockelektrode bezeichnet.

Die Elektrodenleitung 30 ist eine linksventrikuläre Elektrodenleitung an deren distalem Ende eine linksventrikuläre Spitzenelektrode LV Tip 34 sowie in deren Nähe eine linksventrikuläre Ringelektrode LV Ring 32 angeordnet ist. Außerdem trägt die linksventrikuläre Elektrodenleitung 30 eine nicht näher bezeichnete, aber in Figur 2 dargestellte linksventrikuläre Schockwendel zur Abgabe von Defibrillationsschocks an den linken Ventrikel. Die linksventrikuläre Elektrodenleitung 30 wird vom rechten Atrium 26 des Herzens 12 aus über den Koronarsinus in eine von diesem abzweigende Lateralvene geführt und wird deswegen auch als Koronarsinuselektrodenleitung oder CS-Elektrodenleitung bezeichnet.

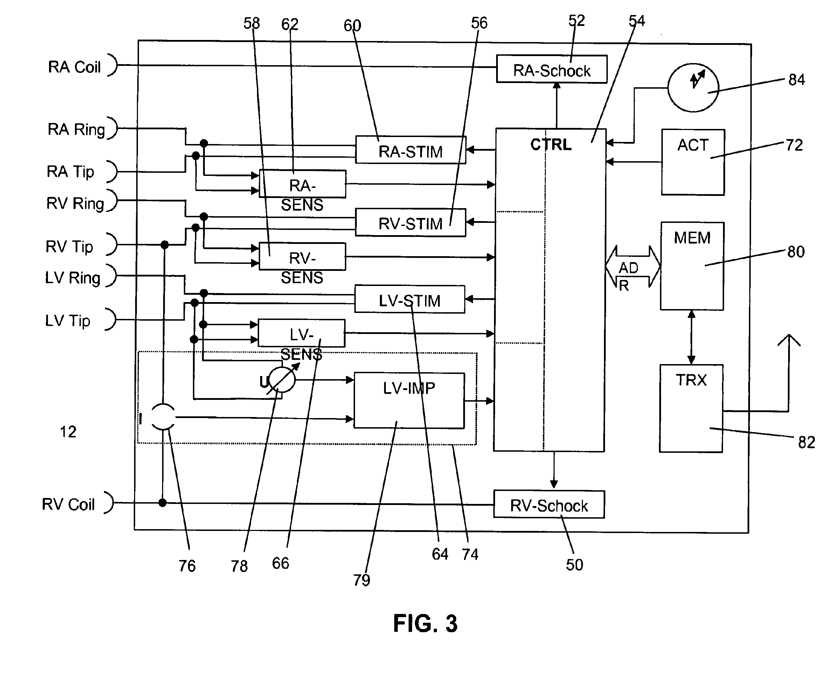

In Figur 3 sind die Hauptbestandteile des Herzstimulators 10 dargestellt. Auf der linken Seite sind die elektrischen Anschlüsse für die verschiedenen Elektroden 18, 20, 22, 24, 32, 34, 38 und 40 dargestellt. Die Schockelektroden 38 und 40 sind jeweils mit einem rechtsventrikulären Schockimpulsgenerator 50 bzw. SVC-Schockgenerator 52 verbunden. Beide Schockgeneratoren 50 und 52 sind mit einer Stimulationssteuereinheit 54 verbunden, die die beiden Schockimpulsgeneratoren 50 und 52 bei Bedarf zur Erzeugung und Abgabe eines Defibrillationsschocks ansteuert.

Der Anschluss für die rechtsventrikuläre Tippelektrode RV Tip sowie der Anschluss für die rechtsventrikuläre Ringelektrode RV Ring sind jeweils sowohl mit einer rechtsventrikulären Stimulationseinheit 56 als auch mit einer rechtsventrikulären Sensingeinheit 58 verbunden. Sowohl die rechtsventrikuläre Stimulationseinheit 56 als auch die rechtsventrikuläre Sensingeinheit 58 sind jeweils mit der Stimulationssteuereinheit 54 verbunden.

Die rechtsventrikuläre Stimulationseinheit 56 ist dazu ausgebildet, auf ein Ansteuersignal der Stimulationssteuereinheit 54 hin einen rechtsventrikulären Stimulationsimpuls zu erzeugen und im Anschluss für die rechtsventrikuläre Ringelektrode die rechtsventrikuläre Tippelektrode abzugeben. Alternativ ist es auch möglich, dass das Gehäuse 42 des Herzstimulators 10 eine neutrale Elektrode bildet und die rechtsventrikuläre Stimulationseinheit 56 mit dem Anschluss für die rechtsventrikuläre Ringelektrode RV Tip und dem Gehäuse 42 als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, so dass er nicht wie ein Defibrillationsschock auf einen Schlag das vollständige Herzgewebe (Myokard) einer Herzkammer erregt, sondern nur die Herzmuskelzellen in unmittelbarer Umgebung der rechtsventrikulären Spitzenelektrode 18. Diese Erregung breitet sich dann durch natürliche Reizleitung über den gesamten Ventrikel weiter aus und sorgt so für eine stimulierte Kontraktion des Ventrikels.

Die rechtsventrikuläre Sensingeinheit 58 ist dazu ausgebildet, an dem Anschluss für die rechtsventrikuläre Ringelektrode RV Ring und die rechtsventrikuläre Tippelektrode RV Tip anliegende elektrische Potentiale zunächst durch einen Eingangsverstärker zu verstärken und zu filtern. Weiterhin ist die rechtsventrikuläre Sensingeinheit ausgebildet, den Verlauf der an ihren Eingängen anliegenden elektrischen Signale derart auszuwerten, dass die rechtsventrikuläre Sensingeinheit 58 selbsttätig eine natürliche (intrinsische), d. h. selbsttätige Kontraktion des rechten Ventrikels detektiert. Dies kann beispielsweise dadurch geschehen, dass der Verlauf des an den Eingängen der rechtsventrikulären Sensingeinheit 58 anliegenden Signals mit einem Schwellwert verglichen wird. Typischerweise ist die größte Amplitude des Signals in Form der sogenannten R Zacke kennzeichnend für eine natürliche Kontraktion des rechten Ventrikels, die durch Schwellwertvergleich detektiert werden kann. Die rechtsventrikuläre Sensingeinheit 58 gibt daraufhin ein entsprechendes, eine natürliche Kontraktion des rechten Ventrikels anzeigendes Ausgangssignal an die Stimulationssteuereinheit 54 aus.

In analoger Weise sind der Anschluss für die rechtsatriale Tippelektrode und der Anschluss für die rechtsatriale Ringelektrode sowohl mit einer rechtsatrialen Stimulationseinheit 60 als auch mit einer rechtsatrialen Sensingeinheit 62 verbunden, die jeweils ihrerseits wiederum mit der Stimulationssteuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatrialen Stimulationsimpulse können dabei eine andere Impulsstärke besitzen, als die rechtsventrikulären Stimulationsimpulse. Die rechtsatriale Sensingeinheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums kennzeichnet. Detektiert die rechtsatriale Sensingeinheit 62 eine entsprechende P-Welle, erzeugt sie ein Ausgangssignal und gibt dieses an die Stimulationssteuereinheit 54 weiter, welches eine natürliche Kontraktion des rechten Atriums kennzeichnet.

In gleicher Weise sind auch der Anschluss für die linksventrikuläre Tippelektrode LV Tip und der Anschluss für die linksventrikuläre Ringelektrode LV Ring mit einer linksventrikulären Stimulationseinheit 64 und einer linksventrikulären Sensingeinheit 66 verbunden. Die linksventrikuläre Stimulationseinheit 64 und die linksventrikuläre Sensingeinheit 66 sind ebenso mit der Stimulationssteuereinheit 54 verbunden. Beide funktionieren ähnlich wie die bereits beschriebenen Stimulationseinheiten 56 und 60 und Sensingeinheiten 58 und 62.

Als weiterer Bestandteil des Herzstimulators 10 ist ein Aktivitätssensor 72 mit der Stimulationssteuereinheit 54 verbunden. Der Aktivitätssensor 72 ist dazu ausgebildet, ein von der körperlichen Aktivität eines Patienten abhängiges Signal, zum Beispiel ein Bewegungssignal, zu erfassen und ein entsprechendes, die körperliche Aktivität des Patienten anzeigendes Signal an die Stimulationssteuereinheit 54 auszugeben. Dies erlaubt es, dass die Stimulationssteuereinheit 54 das Timing der Stimulationsimpulse an den Bedarf des Patienten (hämodynamischen Bedarf) anpasst.

Weiterhin ist eine Impedanzmesseinheit 74 vorgesehen, über die ein gepulster Messstrom mittels einer Stromquelle 76 über die rechtsventrikuläre Tippelektrode (Anschluss RV Tip) und die rechtsventrikuläre Schockwendel (Anschluss RV Coil) abgegeben werden kann. Eine Spannungsmesseinheit 78 misst den daraus resultierenden Spannungsabfall. Eine Impedanzauswerteeinheit 79 ist ausgebildet, hieraus ein Impedanzsignal zu bilden. Zur Abgabe dieses Impedanzsignals ist sie mit der Stimulationssteuereinheit 54 verbunden. Mit Hilfe der Impedanzmesseinheit 54 können in vorteilhafter Weise solche Größen wie die Ejektionsfraktion (EF) oder - bei entsprechend anderer Elektrodenkonfiguration - der mitrale Rückfluss (MR) bestimmt werden und für eine Optimierung des Timings der Stimulationsimpulse durch die Stimulationssteuereinheit 54 berücksichtigt werden.

Weiterhin umfasst der Herzstimulator 10 eine Speichereinheit 80, die mit der Stimulationssteuereinheit 54 verbunden ist und es erlaubt, von der Stimulationssteuereinheit 54 erzeugte oder ausgewertete Signale zu speichern. Andererseits erlaubt es die Speichereinheit 80, Steuerprogramme für die Stimulationssteuereinheit 54 in veränderbarer Form zu speichern. Des weiteren ist die Stimulationssteuereinheit 54 mit einem Zeitgeber 84 verbunden.

Die Speichereinheit 80 ist mit einer Telemetrieeinheit 82 verbunden, die es erlaubt, in der Speichereinheit 80 abgelegte Daten drahtlos an das externe Gerät 100 zu übertragen oder Programmierbefehle seitens des externen Geräts 100 zu dem Herzstimulator 10 zu übertragen und in der Speichereinheit 80 zu speichern.

Als Dreikammerherzstimulator/Kardioverter/Defibrillator ist der Herzstimulator 10 in der Lage, eine Stimulation des rechten Atriums, des rechten Ventrikels und des linken Ventrikels oder auch nur einer oder zweier dieser Herzkammern in an sich bekannter Weise durchzuführen. Dies schließt insbesondere die Stimulation einer jeweiligen Herzkammer im Demand-Modus ein, in der Stimulationsimpulse nur dann an die jeweilige Herzkammer abgegeben werden, falls in einem vorangehenden, jeweiligen Escape-Intervall seitens der jeweiligen Sensingeinheit keine intrinsische Kontraktion der jeweiligen Herzkammer erfasst wird. Damit ist der Herzschrittmacher in der Lage, die bekannten rechtsventrikulären Stimulationsmodi wie VVI, VVD oder DDD durchzuführen. Weiterhin ist der Herzschrittmacher vorzugsweise in der Lage, für die Stimulation des rechten Ventrikels unterschiedlich lange Escape-Intervalle vorzusehen, je nachdem ob im jeweiligen vorangegangenen Escape-Intervall eine intrinsische Kontraktion des Ventrikels detektiert wurde oder nicht. Liegen regelmäßig intrinsische Kontraktionen des Ventrikels vor, wird dem längeren rechtsatrialen Escape-Intervall der Vorzug gegeben, um dem Eigenrhythmus des Herzens den Vorzug zu geben. Dieses verlängerte rechtsventrikuläre Escape-Intervall ist etwas länger, als physiologisch angemessen. Ein kürzeres, physiologisch angemessenes rechtsventrikuläres Escape-Intervall wird immer dann automatisch gewählt, wenn keine rechtsventrikulären Eigenaktionen des Herzens detektiert werden, so dass der rechte Ventrikel regelmäßig stimuliert wird. Bekannt sind in diesem Zusammenhang eine Frequenzhysterese, bei der ein entsprechendes Intervall zwischen aufeinanderfolgenden rechtsventrikulären Ereignissen verlängert bzw. verkürzt wird und eine AV-Zeit-Hysterese, bei der das Intervall zwischen einem rechtsatrialen Ereignis und dem nächsten vorgesehenen rechtsventrikulären Stimulationsimpuls verlängert bzw. verkürzt wird. Als Ereignis wird in diesem Zusammenhang eine jeweilige Kontraktion einer Herzkammer bezeichnet. Ein natürliches Ereignis bezeichnet dementsprechend eine selbsttätige, natürliche (intrinsische) Kontraktion einer jeweiligen Herzkammer und ein stimuliertes Ereignis einer auf einen entsprechenden Stimulationsimpuls zurückgehende Kontraktion der jeweiligen Herzkammer.

Die im vorliegenden Zusammenhang wichtigste Eigenschaft des Herzstimulators 10 besteht in seiner Fähigkeit, durch Stimulation des rechten Ventrikels und des linken Ventrikels eine kardiale Resynchronisationstherapie (CRT) durchzuführen. Der entsprechende Stimulationsmodus wird als biventrikulärer Stimulationsmodus bezeichnet und wird von der Stimulationssteuereinheit 54 gesteuert. Die Optimierung des Timings der Stimulationsimpulse für den rechten und den linken Ventrikel erfolgt dabei in grundsätzlich bekannter Weise beispielsweise unter Berücksichtigung einer jeweils ermittelten Ejektionsfraktion (siehe oben) oder des mitralen Rückflusses (siehe ebenfalls oben sowie in EP 1930045 A1).

Für dieses Timing ist insbesondere eine interventrikuläre Verzögerungszeit (VV-Intervall) von Bedeutung, d. h. die Zeit, mit der ein rechter Stimulationsimpuls und ein linker Stimulationsimpuls (sofern sie im Demand-Modus nicht inhibiert werden) aufeinander folgen. Diese Zeit kann größer als 0 sein, so dass der linke Stimulationsimpuls dem rechten Stimulationsimpuls nachfolgt. Die interventrikuläre Verzögerungszeit kann 0 sein, was bedeutet, dass ein rechtsventrikulärer Stimulationsimpuls und ein linksventrikulärer Stimulationsimpuls durch gleichzeitige Ansteuerung der rechtsventrikulären Stimulationseinheit 56 in der linksventrikulären Stimulationseinheit 64 durch die Stimulationssteuereinheit 54 gleichzeitig abgegeben werden. Die interventrikuläre Verzögerungszeit kann auch kleiner 0 sein, was bedeutet, dass ein linksventrikulärer Stimulationsimpuls vor der Abgabe des zugehörigen rechtsventrikulären Stimulationsimpulses abgegeben wird.

Die biventrikuläre Stimulation kann auch im sogenannten VP-Supressionsmodus erfolgen, in dem rechtsventrikuläre Stimulationsimpulse zwar getimt werden, dann aber tatsächlich nicht abgegeben werden, auch wenn sie nicht durch ein vorangegangenes intrinsisches Ereignis im Demand-Modus inhibiert werden. Ausführungsvarianten eines solchen, für den hier beschriebenen Herzstimulator geeigneten VP-Supressionsmodus sind ausführlich in der Anmeldung US 2008/0009910 A1 beschrieben.

Der Herzstimulator 10 ist ausgebildet, automatisch von der Stimulationssteuereinheit 54 gesteuert zwischen einem rechtsventrikulären Stimulationsmodus und einem biventrikulären Stimulationsmodus wechselseitig umzuschalten.

Die Stimulationssteuereinheit 54 führt dieses Umschalten so durch, dass die Stimulationssteuereinheit zunächst ein die Dauer (bezogen auf die graphische Darstellung eines EKG auch als "Breite" bezeichnet) eines QRS-Komplexes anzeigendes Signal erzeugt. Der mit QRS-Komplex bezeichnete Signalabschnitt eines Kardiogramms ist grundsätzlich bekannt. Im Folgenden wird dieser Signalabschnitt als QRS-Signalabschnitt bezeichnet. Das entsprechende, die Dauer dieses QRS-Signalabschnittes anzeigenden Signals könnte die Stimulationssteuereinheit 54 beispielsweise durch Auswerten eines intrakardial erfassten Elektrogramms (IEGM) wie es beispielsweise über die rechtsventrikuläre Sensingeinheit 58 aufgenommen wird, bestimmt werden. Vorzugsweise bestimmt die Stimulationssteuereinheit 54, dass die Dauer des QRS-Signalabschnittes eigene Signal jedoch durch Bestimmen der natürlichen, interventrikulären Überleitungszeit (intrinsisches VV-Intervall), indem die Stimulationssteuereinheit 54 den zeitlichen Abstand zwischen einer von der rechtsventrikulären Sensingeinheit 58 erfassten Kontraktion des rechten Ventrikels (intrinsisches rechtsventrikuläres Ereignis) und einer von der linksventrikulären Sensingeinheit 66 erfassten, der rechtsventrikulären Kontraktion kausal zuzuordnenden Kontraktion des linken Ventrikels (linksventrikuläres Ereignis) bestimmt. Alternative bevorzugte Ausführungsvarianten der Stimulationssteuereinheit 54 zur Bestimmung des die Dauer des QRS-Signalabschnitts anzeigenden Signals bestehen darin, dass die Stimulationssteuereinheit 54 dieses Signal als die atrio-ventrikuläre Überleitungszeit bestimmt, welches die Dauer zwischen einer von der rechtsatrialen Sensingeinheit 62 erfassten Kontraktion des rechten Atriums und einer von der rechtsventrikulären Sensingeinheit 58 oder der linksventrikulären Sensingeinheit 66 erfassten Kontraktion des rechten bzw. linken Ventrikels ist.

Die Stimulationssteuereinheit 54 ist ausgebildet, das so bestimmte, die Dauer eines QRS-Signalabschnitts anzeigende Signal mit einem Vergleichswert zu vergleichen. Hierzu weist die Stimulationssteuereinheit 54 eine entsprechende Vergleichseinrichtung auf.

Gemäß einer bevorzugten Ausführungsvariante sind zwei unterschiedliche Vergleichswerte vorgesehen, von denen ein erster Vergleichswert als Vergleichskriterium für das Umschalten vom biventrikulären Stimulationsmodus in den rechtsventrikulären Stimulationsmodus dient und ein zweiter Vergleichswert als Vergleichskriterium für das Umschalten vom rechtsventrikulären Stimulationsmodus in den biventrikulären Stimulationsmodus dient. Der erste Vergleichswert entspricht der vergleichsweise kürzeren Dauer des QRS-Signalabschnittes als Referenzwert und der zweite Vergleichswert entspricht einer vergleichsweise längeren Dauer des QRS-Signalabschnitts als Referenzwert. Auf diese Weise neigt die Stimulationssteuereinheit 54 dazu, den jeweils anliegenden Stimulationsmodus etwas länger beizubehalten.

Dem gleichen Zweck dient eine bevorzugte Ausführungsvariante der Stimulationssteuereinheit 54, die darauf hinausläuft, dass diese ein Umschalten von jeweils einem Stimulationsmodus in den jeweils anderen Stimulationsmodus erst vornimmt, wenn der jeweilige Vergleichswert eine vorgegebene Anzahl von mehreren Herzzyklen über- bzw. unterschritten wird.

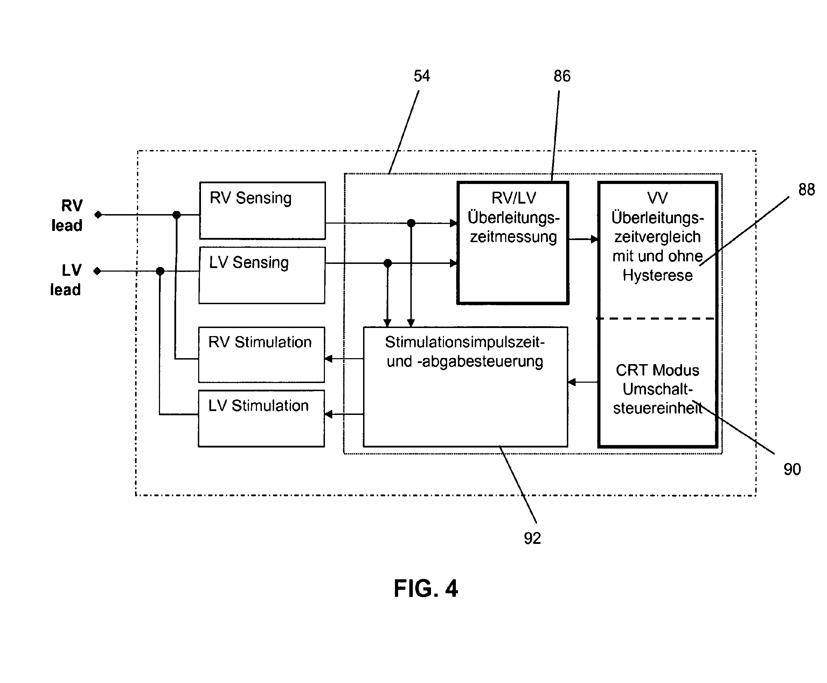

Der Erläuterung dieser Funktionsweise der Stimulationssteuereinheit 54 dient die Abbildung in Figur 4, die neben den relevanten Stimulationseinheiten und Sensingeinheiten folgende Bestandteile der Stimulationssteuereinheit 54 zeigt: eine VV-Überleitungszeit-Messeinheit 86, eine VV-Vergleichseinheit 88, einen Stimulationsmodusumschalter 90 und eine Stimulationsimpulszeit- und -abgabesteuereinheit 92. Die VV-Überleitungszeit-Messeinheit 86 wirkt mit dem Zeitgeber 84 zusammen um auf die zuvor beschriebene Weise die intrinsische VV-Überleitungszeit zu bestimmen.

Das Beispiel in Figur 4 beruht auf der Ausführungsvariante gemäß der die Stimulationssteuereinheit 54 ausgebildet ist, das die Dauer des QRS-Signalabschnitts anzeigende Signal proportional zur intrinsischen interventrikulären Überleitungszeit zu bilden.

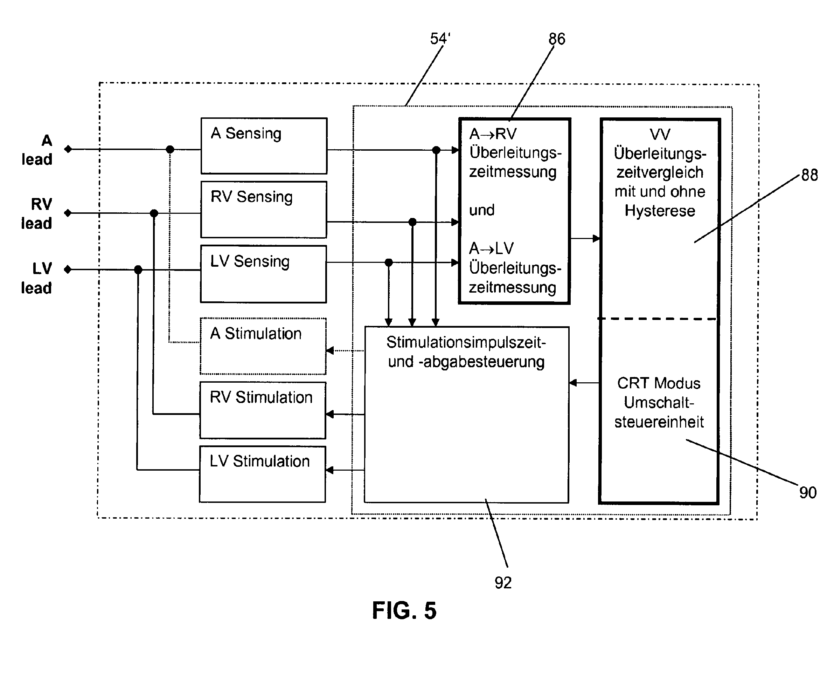

Figur 5 zeigt eine alternative Ausführungsvariante, gemäß der die Stimulationssteuereinheit 54' ausgebildet ist, das die Dauer des QRS-Abschnitts anzeigende Signals proportional zur atrio-ventrikulären Überleitungszeit zu bilden, und zwar entweder proportional zur Überleitungszeit vom rechten Atrium zum rechten Ventrikel oder zur Überleitungszeit vom rechten Atrium zum linken Ventrikel. Entsprechend ist in dem Ausführungsbeispiel gemäß Figur 5 die Stimulationssteuereinheit 54' auch mit allen drei Elektrodenleitungen, nämlich der rechtsatrialen Elektrodenleitung 14, der rechtsventrikulären Elektrodenleitung 16 und der linksventrikulären Elektrodenleitung 30 verbunden.

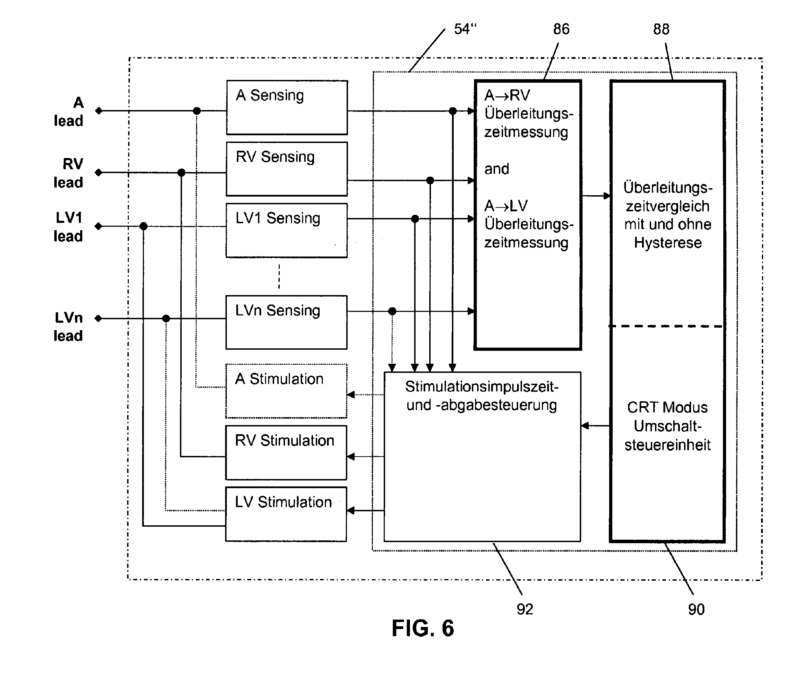

Figur 6 zeigt ein Ausführungsbeispiel, das darauf hinausläuft, dass nicht nur eine einzige linksventrikuläre Elektrodenleitung wie die Elektrodenleitung 30 vorgesehen sein kann, sondern dass der Herzstimulator auch ausgebildet sein kann, mit einer Vielzahl von linksventrikulären Elektrodenleitungen verbunden zu werden.

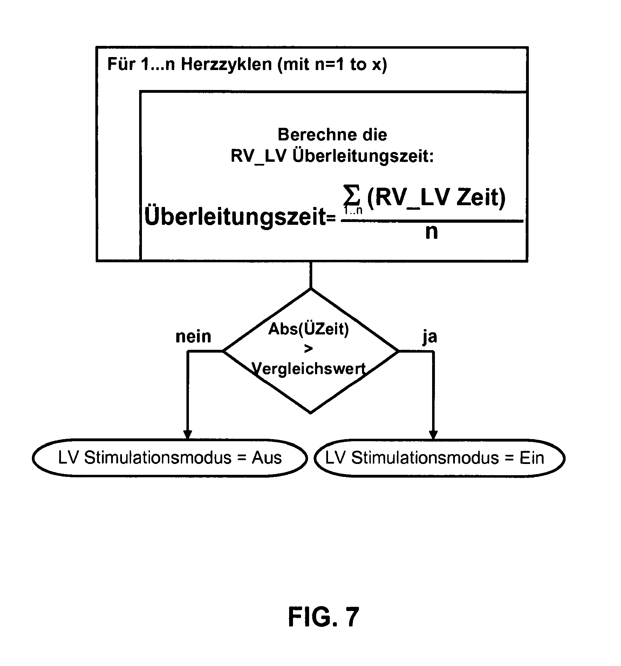

Figur 7 zeigt schließlich in einem Flussdiagramm, wie die Stimulationssteuereinheit 54 die Dauer der intrinsischen interventrikulären Überleitungszeit (intrinsisches VV-Interval) bestimmt und wie anschließend die Umschaltung in den einen oder anderen Stimulationsmodus erfolgt. Zunächst wird der Mittelwert der interventrikulären Überleitungszeit über mehrere Herzzyklen gebildet und anschließend wird der Absolutwert dieser so gebildete Mittelwert (Ü-Zeit) mit einem einzigen Vergleichswert verglichen. Überschreitet der so gebildete Mittelwert der Überleitungszeit diesen Vergleichswert, schaltet die Stimulationssteuereinheit 54 den Herzstimulator 10 in den biventrikulären Stimulationsmodus, in dem auch der linke Ventrikel stimuliert wird. Ist die gemittelte Überleitungszeit hingegen kleiner als der Vergleichswert, schaltet die Stimulationssteuereinheit 54 den Herzstimulator 10 in den rechtsventrikulären Stimulationsmodus, in dem der linke Ventrikel nicht stimuliert wird.

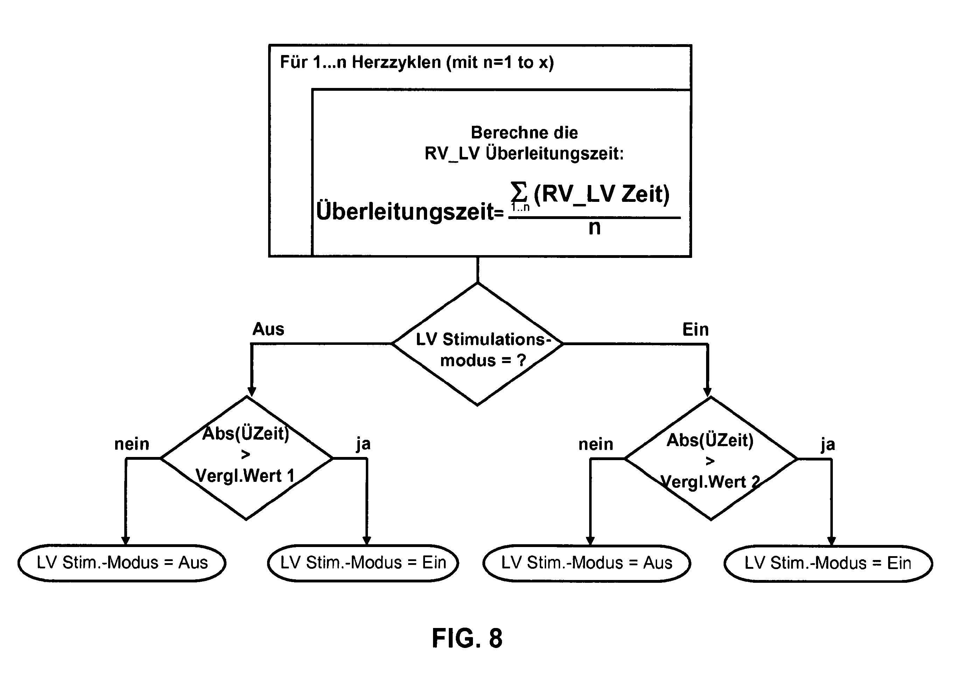

Figur 8 ist ein Flussdiagramm einer bevorzugten Ausführungsvariante bei der zwei unterschiedliche Vergleichswerte vorgesehen sind, mit denen der Absolutwert des über n-Herzzyklen gebildeten Mittelwertes der interventrikulären Überleitungszeit verglichen wird, je nachdem, ob sich der Herzstimulator 10 gerade in seinem biventrikulären Stimulationsmodus befindet (rechter Zweig, Vergleich mit dem zweiten Vergleichswert) oder in dem rechtsventrikulären Stimulationsmodus (Vergleich mit dem ersten Vergleichswert).

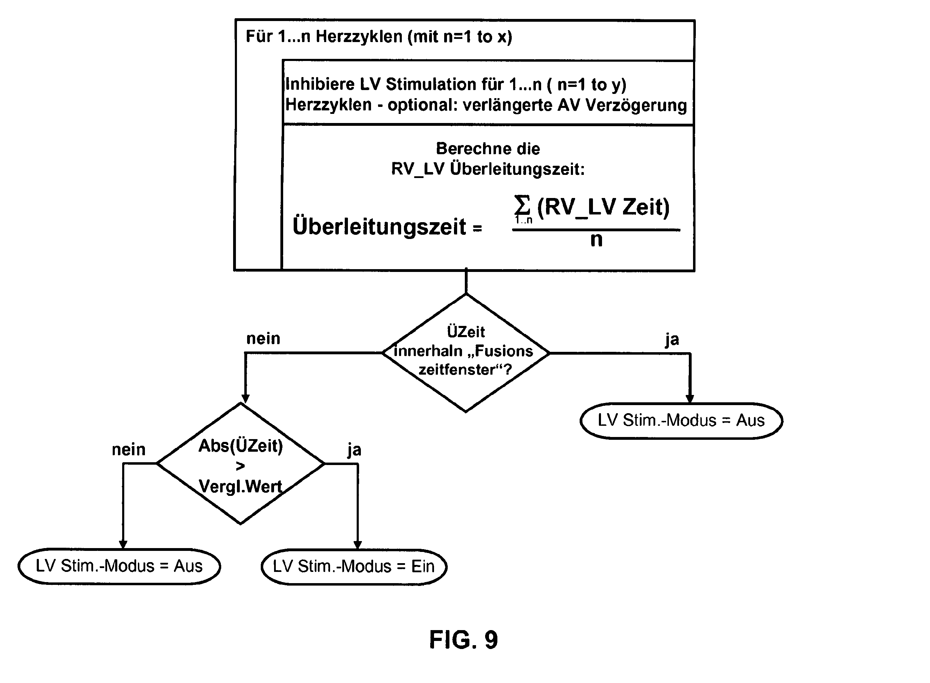

Figur 9 ist schließlich ein Flussdiagramm, welches darstellt, wie die über n-Herzzyklen bei deaktivierter linksventrikulärer Stimulation oder unter Anwendung einer verlängerten AV-Zeit gemittelte interventrikuläre Übergangszeit zunächst mit einem Vergleichsintervall namens "Fusionszeitfenster" verglichen wird. Liegt die gemittelte, interventrikuläre Überleitungszeit in diesem Vergleichszeitfenster, schaltet die Stimulationssteuereinheit 54 den Herzstimulator 10 in jedem Fall in den rechtsventrikuiären Stimulationsmodus. Das Fusionszeitfenster ist dabei ein Zeitfenster, welches um das Ende eines jeweiligen linksventrikulären Escape-Intervalls herumgelegt wird. Das linksventrikuläre Escape-Intervall ist das Intervall, an dessen Ende ein linksventrikulärer Stimulationsimpuls ausgelöst wird, sofern dieser nicht durch ein zuvor auftretendes linksventrikuläres natürliches Ereignis inhibiert wird. Das linksventrikuläre Escape-Intervall wird üblicherweise mit einem rechtsatrialen Ereignis gestartet. Das Fusionszeitfenster ist so gewählt, dass innerhalb des Fusionszeitfensters die Gefahr besteht, dass ein linksventrikulärer Stimulationsimpuls zu einem Zeitpunkt abgegeben wird, zu dem auch eine natürliche linksventrikuläre Kontraktion auftritt, so dass es zu einem sogenannten Fusionsereignis kommt.

Nur wenn der angesprochene Vergleich der gemittelten interventrikulären Überleitungszeit ergeben hat, dass diese außerhalb des Fusionszeitfensters endet, führt die Stimulationssteuereinheit 54 den zuvor bereits beschriebenen Vergleich der gemittelten interventrikulären Überleitungszeit mit einem Vergleichswert durch und schaltet je nach Ausgang des Vergleichs entweder in den biventrikulären Stimulationsmodus oder in den rechtsventrikulären Stimulationsmodus. Die in Figur 9 dargestellte Ausführungsvariante stellt den Fall dar, in dem die gemittelte interventrikuläre Überleitungszeit nur mit einem Vergleichswert verglichen wird. Analog zu der in Figur 8 dargestellten Ausführungsvariante kann dieser Vergleich auch mit zwei unterschiedlichen Vergleichswerten durchgeführt werden.

Zusätzlich oder alternativ kann der Herzstimulator ausgebildet sein, die Umschaltung zwischen rechtsventrikulärem und biventrikulärem Stimulationsmodus in Abhängigkeit von der ermittelten Ejektionsfraktion oder dem bestimmten mitralen Rückfluss oder von beiden Größen derart durchzuführen, dass von einer rechtsventrikulären Stimulation zu einer biventrikulären Stimulation umgeschaltet wird, wenn entweder die gemessene Ejektionsfraktion einen programmierbaren Schwellwert unterschreitet oder der mitrale Rückfluss einen programmierbaren Schwellwert überschreitet. Werden beide Parameter vom Herzstimulator gleichzeitig ausgewertet, erfolgt die Umschaltung von einem rechtsventrikulärem Modus in einen biventrikulären Moduls wenn entweder die Ejektionsfraktion den programmierten Schwellwert unterschreitet oder der mitrale Rückfluss den programmierten Schwellwert überschreitet. Die Umschaltung von einem biventrikulären in einen rechtsventrikulären Stimulationsmodus erfolgt dann, wenn die gemessenen Ejektionsfraktion über einem programmierten zweiten (Hysterese) Schwellwert für die Ejektionsfraktion liegt und der mitrale Rückfluss gleichzeitig unter einem programmierten zweiten (Hysterese) Schwellwert für den mitralen Rückfluss liegt.

**Bezugszeichenliste**

| Bezugszeichen | Bedeutung |
|---|---|
| 10 | Herzstimulator |
| 100 | externes Gerät |
| 110 | Servicecenter |
| 120 | Ärzteteam |
| 11 | standardisierte Kontaktbuchsen in einem Header (Anschlussgehäuse) |
| 12 | Herz |
| 14 | rechtsatriale Elektrodenleitung |
| 16 | rechtsventrikuläre Elektrodenleitung |
| 18 | rechtsventrikuläre Spitzenelektrode RV Tip |
| 20 | rechtsventrikuläre Ringelektrode RV Ring |
| 22 | atriale Spitzenelektrode RA Tip |
| 24 | atriale Ringelektrode RA Ring |
| 26 | rechtes Atrium |
| 28 | rechter Ventrikel |
| 30 | linksventrikuläre Elektrodenleitung |
| 32 | linksventrikuläre Ringelektrode LV Ring |
| 34 | linksventrikuläre Spitzenelektrode LV Tip |
| 38 | rechtsventrikuläre Schockwendel RV Schock |
| 40 | Schockwendel |
| 42 | Gehäuse |
| 50 | rechtsventrikulärer Schockimpulsgenerator |
| 52 | SVC-Schockimpulsgenerator |
| 54 | Stimulationssteuereinheit |
| 56 | rechtsventrikuläre Stimulationseinheit |
| 58 | rechtsventrikuläre Sensingeinheit |
| 60 | rechtsatriale Stimulationseinheit |
| 62 | rechtsatriale Sensingeinheit |
| 64 | linksventrikuläre Stimulationseinheit |
| 66 | linksventrikuläre Sensingeinheit |
| 72 | Aktivitätssensor |
| 74 | Impedanzmesseinheit |
| 76 | Stromquelle |
| 78 | Spannungsmesseinheit |
| 79 | Impedanzauswerteeinheit |
| 80 | Speichereinheit |
| 82 | Telemetrieeinheit |
| 84 | Zeitgeber |
| 86 | VV-Überleitungszeit-Messeinheit |
| 88 | VV-Vergleichseinheit |
| 90 | Stimulationsmodusumschalter |
| 92 | Stimulationsimpulszeit- und -abgabesteuereinheit |

## Patentansprüche

1. Implantierbarer Herzstimulator (10) mit einer rechtsatrialen Sensingeinheit (62), die einen Eingang aufweist, der mit einer rechtsatrialen Elektrodenleitung (14) verbunden oder zu verbinden ist, und die ausgebildet ist, ein an ihrem Eingang anliegendes elektrisches Eingangssignal derart auszuwerten, dass die rechtsatriale Sensingeinheit (62) wenigstes ein für eine Kontraktion eines rechten Atriums typisches Signalmerkmal detektiert und ein entsprechendes Ausgangssignal erzeugt,
wenigstens einer rechtsventrikulären Sensingeinheit (58), die einen Eingang aufweist, der mit einer rechtsventrikulären Elektrodenleitung (16) verbunden oder zu verbinden ist, und die ausgebildet ist, ein an ihrem Eingang anliegendes elektrisches Eingangssignal derart auszuwerten, dass die rechtsventrikuläre Sensingeinheit wenigstes ein für eine Kontraktion eines rechten Ventrikels typisches Signalmerkmal detektiert und ein entsprechendes Ausgangssignal erzeugt,
wenigstens einer rechtsventrikulären Stimulationseinheit (56), die einen Ausgang aufweist, der mit einer rechtsventrikulären Elektrodenleitung (16) verbunden oder zu verbinden ist, und die ausgebildet ist, auf ein Steuersignal hin einen rechtsventrikulären Stimulationsimpuls zu erzeugen und über den Ausgang abzugeben,
wenigstens einer linksventrikulären Sensingeinheit (66), die einen Eingang aufweist, der mit einer linksventrikulären Elektrodenleitung (30) verbunden oder zu verbinden ist, und die ausgebildet ist, ein an ihrem Eingang anliegendes elektrisches Eingangssignal derart auszuwerten, dass die linksventrikuläre Sensingeinheit wenigstes ein für eine Kontraktion eines linken Ventrikels typisches Signalmerkmal detektiert und ein entsprechendes Ausgangssignal erzeugt,
wenigstens einer linksventrikulären Stimulationseinheit (64), die einen Ausgang aufweist, der mit einer linksventrikulären Elektrodenleitung (30) verbunden oder zu verbinden ist, und die ausgebildet ist, auf ein Steuersignal hin einen linksventrikulären Stimulationsimpuls zu erzeugen und über den Ausgang abzugeben,
sowie einer Stimulationssteuereinheit (54), die mit der rechtsventrikulären Sensingeinheit (58), der linksventrikulären Sensingeinheit (66), der rechtsventrikulären Stimulationseinheit (56) und der linksventrikulären Stimulationseinheit (64) verbunden und ausgebildet ist, Ausgangssignale der Sensingeinheiten (58, 66) zu verarbeiten und Steuersignal für die Stimulationseinheiten (56, 64) zu erzeugen,
wobei die Stimulationssteuereinheit (54) ausgebildet ist, ihren Betriebsmodus zwischen wenigstens einem rechtsventrikulären Stimulationsmodus, in dem keine linksventrikuläre Stimulationsimpulse auslösende Steuersignale an die linksventrikuläre Stimulationseinheit ausgegeben werden, und einem biventrikulären Stimulationsmodus wechselseitig umzuschalten, wobei die Stimulationssteuereinheit weiter ausgebildet ist, ein Umschalten in Abhängigkeit eines die Dauer eines jeweils aktuellen QRS-Signalabschnittes eines bestimmten oder beliebigen der elektrischen Eingangssignale anzeigenden Signals vorzunehmen,
**dadurch gekennzeichnet, dass**
die Stimulationssteuereinheit ausgebildet ist in den biventrikulären Stimulationsmodus zu schalten, wenn ein Vergleich des die Dauer eines jeweils aktuellen QRS-Signalabschnittes anzeigenden Signals mit einem ersten Vergleichswert ergibt, dass die Dauer des jeweils aktuellen QRS-Signalabschnittes länger ist als ein durch den ersten Vergleichswert repräsentierter erster Referenzwert, und in den rechtsventrikulären Stimulationsmodus schaltet, wenn ein Vergleich des die Dauer eines jeweils aktuellen QRS-Signalabschnittes anzeigenden Signals mit einem zweiten Vergleichswert ergibt, dass die Dauer des jeweils aktuellen QRS-Signalabschnittes kürzer ist als ein durch den zweiten Vergleichswert repräsentierter zweiter Referenzwert,
wobei die Stimulationssteuereinheit (54) ausgebildet ist, das die Dauer eines QRS-Signalabschnitts anzeigende Signal so zu bestimmen, dass das die Dauer eines QRS-Signalabschnitts anzeigende Signal den zeitlichen Abstand zwischen einer von der rechtsatrialen Sensingeinheit (62) erfassten Kontraktion des rechten Atriums und einer von der linksventrikulären Sensingeinheit (66) erfassten, der rechtsatrialen Kontraktion kausal zuzuordnenden Kontraktion des linken Ventrikels widerspiegelt.

2. Implantierbarer Herzstimulator nach Anspruch 1, **dadurch gekennzeichnet, dass** der erste und der zweite Vergleichswert sowie der erste und der zweite Referenzwert jeweils identisch sind.

3. Implantierbarer Herzstimulator nach Anspruche 1 oder 2, **dadurch gekennzeichnet, dass** die Stimulationssteuereinheit (54) ausgebildet ist, in dem rechtsventrikulären Stimulationsmodus die Abgabe rechtsventrikulärer Stimulationsimpulse zu unterdrücken (Vp Suppression).

4. Implantierbarer Herzstimulator nach einem der Ansprüche 1 bis 3, **dadurch gekennzeichnet, dass** die Stimulationssteuereinheit (54) ausgebildet ist, ein Umschalten von einem zum anderen Stimulationsmodus nach jedem Über- bzw. Unterschreiten des jeweiligen Vergleichswertes vorzunehmen.

5. Implantierbarer Herzstimulator nach einem der Ansprüche 1 bis 3, **dadurch gekennzeichnet, dass** die Stimulationssteuereinheit (54) ausgebildet ist, ein Umschalten von einem zum anderen Stimulationsmodus erst vorzunehmen, wenn das die Dauer eines QRS-Signalabschnitts anzeigende Signal den jeweiligen Vergleichswert für eine jeweils vorgegebene Anzahl von Herzzyklen über bzw. unterschritten hat.

6. Implantierbarer Herzstimulator nach einem der Ansprüche 1 bis 5, **dadurch gekennzeichnet, dass** die Stimulationssteuereinheit (54) ausgebildet ist, in dem biventrikulären Stimulationsmodus regelmäßig ein jeweiliges intrinsische VV-Intervall zu erfassen, das den zeitlichen Abstand zwischen einer von der rechtsventrikulären Sensingeinheit (58) erfassten Kontraktion des rechten Ventrikels und einer von der linksventrikulären Sensingeinheit (66) erfassten, der rechtsventrikulären Kontraktion kausal zuzuordnenden Kontraktion des linken Ventrikels widerspiegelt, und einen linksventrikulären Stimulationszeitpunkt jeweils so zu bestimmen, dass er nach dem Ende des so bestimmten intrinsischen VV-Intervall liegt.

7. Implantierbarer Herzstimulator nach einem der Ansprüche 1 bis 6, **dadurch gekennzeichnet, dass** die Stimulationssteuereinheit (54) mit einer Speichereinheit (80) verbunden und ausgebildet ist, die Zeitpunkte des Umschaltens zwischen den Stimulationsmodi in der Speichereinheit (80) zu Speichern.

8. Implantierbarer Herzstimulator nach Anspruch 7, **dadurch gekennzeichnet, dass** die Speichereinheit (80) mit einer Telemetrieeinheit (82) verbunden ist, die ausgebildet ist in der Speichereinheit (80) gespeicherte Daten drahtlos zu übertragen.

## Claims

1. An implantable heart stimulator (10) comprising
a right-atrial sensing unit (62), which has an input that is connected or is to be connected to a right-atrial electrode line (14), and which is configured to evaluate an electric input signal present at the input thereof in such a way that the right-atrial sensing unit (62) detects at least one signal feature typical for a contraction of a right atrium and generates a corresponding output signal,
further comprising a right-ventricular sensing unit (58), which has an input that is connected or is to be connected to a right-ventricular electrode line (16), and which is configured to evaluate an electric input signal present at the input thereof in such a way that the right-ventricular sensing unit detects at least one signal feature typical for a contraction of a right ventricle and generates a corresponding output signal,
further comprising at least one right-ventricular stimulation unit (56), which has an output that is connected or is to be connected to a right-ventricular electrode line (16), and which is configured, in response to a control signal, to generate a right-ventricular stimulation pulse and to deliver this via the output,
further comprising at least one left-ventricular sensing unit (66), which has an input that is connected or is to be connected to a left-ventricular electrode line (30), and which is configured to evaluate an electric input signal present at the input thereof in such a way that the left-ventricular sensing unit detects at least one signal feature typical for a contraction of a left ventricle and generates a corresponding output signal,
further comprising at least one left-ventricular stimulation unit (64), which has an output that is connected or is to be connected to a left-ventricular electrode line (30), and which is configured to generate, in response to a control signal, a left-ventricular stimulation pulse and to deliver this via the output,
and also comprising a stimulation control unit (54), which is connected to the right-ventricular sensing unit (58), the left-ventricular sensing unit (66), the right-ventricular stimulation unit (56) and the left-ventricular stimulation unit (64) and is configured to process output signals of the sensing units (58, 66) and to generate a control signal for the stimulation units (56, 64),
wherein the stimulation control unit (54) is configured to switch over its operating mode reciprocally between at least one right-ventricular stimulation mode, in which no control signals triggering left-ventricular stimulation pulses are output to the left-ventricular stimulation unit, and a biventricular stimulation mode, wherein the stimulation control unit is also configured to perform a switchover depending on the duration of a current QRS signal portion of a signal indicating a certain one or any of the electric input signals, **characterised in that** the stimulation control unit is configured to switch into the biventricular stimulation mode when a comparison of the signal indicating the duration of a current QRS signal portion with a first comparison value reveals that the duration of the current QRS signal portions is longer than a first reference value represented by the first comparison value, and switches into the right-ventricular stimulation mode when a comparison of the signal indicating the duration of a current QRS signal portion with a second comparison value reveals that the duration of the current QRS signal portion is shorter than a second reference value represented by the second comparison value,
wherein the stimulation control unit (54) is configured to determine the signal indicating the duration of a QRS signal portion such that the signal indicating the duration of a QRS signal portion reflects the time interval between contraction of the right atrium detected by the right-atrial sensing unit (62) and a contraction of the left ventricle detected by the left-ventricular sensing unit (66) and to be assigned causally to the right-atrial contraction.

2. The implantable heart stimulator according to Claim 1, **characterised in that** the first and the second comparison value are identical and also the first and second reference value are identical.

3. The implantable heart stimulator according to Claims 1 or 2, **characterised in that** the stimulation control unit (54) is configured to suppress the delivery of right-ventricular stimulation pulses in the right-ventricular stimulation mode (Vp suppression).

4. The implantable heart stimulator according to one of Claims 1 to 3, **characterised in that** the stimulation control unit (54) is configured to perform a switchover from one stimulation mode to the other once the respective comparison value has been exceeded or undershot.

5. The implantable heart stimulation according to one of Claims 1 to 3, **characterised in that** the stimulation control unit (54) is configured to perform a switchover from one stimulation mode to the other only when the signal indicating the duration of a QRS signal portion has exceeded or undershot the respective comparison value for a predefined number of heart cycles.

6. The implantable heart stimulator according to one of Claims 1 to 5, **characterised in that** the stimulation control unit (54) is configured, in the biventricular stimulation mode, to regularly detect a respective intrinsic VV interval, which reflects the time interval between a contraction of the right ventricle detected by the right-ventricular sensing unit (58) and a contraction of the left ventricle detected by the left-ventricular sensing unit (66) and to be assigned causally to the right-ventricular contraction, and to determine a left-ventricular stimulation time such that it lies after the end of the intrinsic VV interval thus determined.

7. The implantable heart stimulator according to one of Claims 1 to 6, **characterised in that** the stimulation control unit (54) is connected to a memory unit (80) and is configured to store in the memory unit (80) the times of the switchover between the stimulation modes.

8. The implantable heart stimulator according to Claim 7, **characterised in that** the memory unit (80) is connected to a telemetry unit (82), which is configured to wirelessly transfer data stored in the memory unit (80).

## Revendications

1. Stimulateur cardiaque implantable (10) avec une unité de détection d'oreillette droite (62) comportant une entrée reliée ou destinée à être reliée à une ligne d'électrodes d'oreillette droite (14), et conçue pour évaluer un signal d'entrée électrique appliqué à son entrée de manière à ce que l'unité de détection d'oreillette droite (62) détecte au moins une caractéristique de signal typique d'une contraction d'une oreillette droite et produise un signal de sortie correspondant,
au moins une unité de détection de ventricule droit (58) comportant une entrée reliée ou destinée à être reliée à une ligne d'électrodes de ventricule droit (16), et conçue pour évaluer un signal d'entrée électrique appliqué à son entrée de manière à ce que l'unité de détection de ventricule droit détecte au moins une caractéristique de signal typique d'une contraction d'un ventricule droit et produise un signal de sortie correspondant,
au moins une unité de stimulation de ventricule droit (56) comportant une sortie reliée ou destinée à être reliée à une ligne d'électrodes de ventricule droit (16), et conçue pour produire une impulsion de stimulation de ventricule droit en réponse à un signal de commande,
au moins d'une unité de détection de ventricule gauche (66) comportant une entrée reliée ou destinée à être reliée à une ligne d'électrodes de ventricule gauche (30), et conçue pour évaluer un signal d'entrée électrique appliqué à son entrée de manière à ce que l'unité de détection de ventricule gauche détecte au moins une caractéristique de signal typique d'une contraction d'un ventricule gauche et produise un signal de sortie correspondant,
au moins une unité de stimulation de ventricule gauche (64) comportant une sortie reliée ou destinée à être reliée à une ligne d'électrodes de ventricule gauche (16), et conçue pour produire une impulsion de stimulation de ventricule gauche en réponse à un signal de commande,
ainsi qu'une unité de commande de stimulation (54) reliée à l'unité de détection de ventricule droit (58), à l'unité de détection de ventricule gauche (66), à l'unité de stimulation de ventricule droit (56) et à l'unité de stimulation de ventricule gauche (64), et conçue pour traiter des signaux de sortie des unités de détection (58, 66) et pour produire un signal de commande pour les unités de stimulation (56, 64),
dans lequel l'unité de commande de stimulation (54) est conçue pour changer alternativement de mode de fonctionnement, entre au moins un mode de stimulation de ventricule droit, dans lequel aucun signal de commande déclencheur d'impulsions de stimulation n'est délivré à l'unité de stimulation de ventricule gauche, et un mode de stimulation biventriculaire, dans lequel l'unité de commande de stimulation est en outre conçue pour assurer une commutation en fonction d'un signal indiquant la durée d'une section de signal QRS respectivement actuelle d'un signal d'entrée en particulier ou au choix parmi les signaux d'entrée électriques, **caractérisé en ce que** l'unité de commande de stimulation est conçue pour passer dans le mode de stimulation biventriculaire lorsqu'une comparaison du signal indiquant la durée d'une section de signal QRS respectivement actuelle avec une première valeur de comparaison révèle que la durée de la section de signal QRS respectivement actuelle est supérieure à une première valeur de référence représentée par la première valeur de comparaison, et pour passer dans le mode de stimulation de ventricule droit lorsqu'une comparaison du signal indiquant la durée d'une section de signal QRS respectivement actuelle avec une deuxième valeur de comparaison révèle que la durée de la section de signal QRS respectivement actuelle est inférieure à une deuxième valeur de référence représentée par la deuxième valeur de comparaison,
dans lequel l'unité de commande de stimulation (54) est conçue pour déterminer le signal indiquant la durée d'une section de signal QRS de manière à ce que le signal indiquant la durée d'une section de signal QRS reflète l'écart temporel entre une contraction de l'oreillette droite détectée par l'unité de détection d'oreillette droite (62), et une contraction de ventricule gauche à attribuer de façon causale à la contraction de ventricule droit, détectée par l'unité de détection de ventricule gauche (66).

2. Stimulateur cardiaque implantable selon la revendication 1, **caractérisé en ce que** la première et la deuxième valeur de comparaison ainsi que la première et la deuxième valeur de référence sont respectivement identiques.

3. Stimulateur cardiaque implantable selon la revendication 1 ou 2, **caractérisé en ce que** l'unité de commande de stimulation (54) est conçue pour empêcher (Vp suppression) la délivrance d'impulsions de stimulation de ventricule droit dans le mode de stimulation de ventricule droit.

4. Stimulateur cardiaque implantable selon l'une des revendications 1 à 3, **caractérisé en ce que** l'unité de commande de stimulation (54) est conçue pour assurer une commutation d'un mode de stimulation à l'autre, après chaque passage en dessous ou au-dessus de la valeur de comparaison respective.

5. Stimulateur cardiaque implantable selon l'une des revendications 1 à 3, **caractérisé en ce que** l'unité de commande de stimulation (54) est conçue pour assurer une commutation d'un mode de stimulation à l'autre seulement lorsque le signal indiquant la durée d'une section de signal QRS est passé en dessous ou au-dessus de la valeur de comparaison respective pour un nombre respectivement prédéfini de cycles cardiaques.

6. Stimulateur cardiaque implantable selon l'une des revendications 1 à 5, **caractérisé en ce que** l'unité de commande de stimulation (54) est conçue pour détecter régulièrement un intervalle VV intrinsèque respectif dans le mode de stimulation biventriculaire, lequel reflète l'écart temporel entre une contraction du ventricule droit détectée par l'unité de détection de ventricule droit (58), et une contraction de ventricule gauche à attribuer de façon causale à la contraction de ventricule droit, détectée par l'unité de détection de ventricule gauche (66), et pour déterminer un moment de stimulation de ventricule gauche de manière à ce que celui-ci se situe après la fin de l'intervalle VV intrinsèque ainsi déterminé.

7. Stimulateur cardiaque implantable selon l'une des revendications 1 à 6, **caractérisé en ce que** l'unité de commande de stimulation (54) est reliée à une unité de mémoire (80) et conçue pour enregistrer les moments de commutation entre les modes de stimulation dans l'unité de mémoire (80).

8. Stimulateur cardiaque implantable selon la revendication 7, **caractérisé en ce que** l'unité de mémoire (80) est reliée à une unité de télémétrie (82) conçue pour transmettre sans fil des données enregistrées dans l'unité de mémoire (80).
